# EUROPÄISCHE PATENTSCHRIFT

(11) **EP 1 499 366 B1**
(45) Veröffentlichungstag und Bekanntmachung des Hinweises auf die Patenterteilung: **25.07.2007**
(21) Anmeldenummer: 02780963.1
(22) Anmeldetag: 04.09.2002
(51) Int. Cl.: A61L 27/38, A61L 27/18, A61L 31/00, A61F 2/24

(54) **IN-VITRO-VERFAHREN ZUM HERSTELLEN EINER HOMOLOGEN "GESTENTETEN" TISSUE ENGINEERTEN HERZKLAPPE**
IN-VITRO METHOD FOR THE PRODUCTION OF A HOMOLOGOUS STENTED TISSUE-ENGINEERED HEART VALVE
PROCEDE IN-VITRO DE PRODUCTION D'UNE VALVULE CARDIAQUE "STENTEE" HOMOLOGUE ISSUE DU GENIE TISSULAIRE

(30) Priorität: 01.08.2002 DE 10235237
(43) Veröffentlichungstag der Anmeldung: 26.01.2005
(73) Patentinhaber: Universität Zürich, 8006 Zürich (CH)
(72) Erfinder: Covelli, Bruno, 5034 Suhr (CH)
(74) Vertreter: Grünecker, Kinkeldey, Stockmair & Schwanhäusser Anwaltssozietät
(86) Internationale Anmeldenummer: PCT/EP2002/009906
(87) Internationale Veröffentlichungsnummer: WO 2004/018008

(56) Entgegenhaltungen:
- WO-A-01/82840
- DE-A- 19 828 726
- DE-A- 19 919 625
- US-A- 5 855 610
- SODIAN R ET AL: "Tissue engineering of heart valves: in vitro experiences." THE ANNALS OF THORACIC SURGERY. UNITED STATES JUL 2000, Bd. 70, Nr. 1, Juli 2000 (2000-07), Seiten 140-144, XP002244993 ISSN: 0003-4975
- SODIAN R ET AL: "APPLICATION TO STEREOLITHOGRAPHY FOR SCAFFOLD FABRICATION FOR TISSUE ENGINEERED HEART VALVES" ASAIO JOURNAL, J.B.LIPPINCOTT CO.,HAGERSTOWN,MD, US, Bd. 48, Nr. 1, Januar 2002 (2002-01), Seiten 12-16, XP001112831 ISSN: 1058-2916
- HOERSTRUP S P ET AL: "TISSUE ENGINEERING OF A BIOPROSTHETIC HEART VALVE: STIMULATION OF EXTRACELLULAR MATRIX ASSESSED BY HYDROXYPROLINE ASSAY" ASAIO JOURNAL, J.B.LIPPINCOTT CO.,HAGERSTOWN,MD, US, Bd. 45, Nr. 5, September 1999 (1999-09), Seiten 397-402, XP000880997 ISSN: 1058-2916
- HOERSTRUP S P ET AL: "NEW PULSATILE BIOREACTOR FOR IN VITRO FORMATION OF TISSUE ENGINEERED HEART VALVES" TISSUE ENGINEERING, LARCHMONT, NY, US, Bd. 6, Nr. 1, Februar 2000 (2000-02), Seiten 75-79, XP000993059 ISSN: 1076-3279
- ZUEND G ET AL: "TISSUE ENGINEERING: A NEW APPROACH IN CARDIOVASCULAR SURGERY;SEEDING OF HUMAN FIBROBLASTS FOLLOWED BY HUMAN ENDOTHELIAL CELLS ONRESORBABLE MESH" EUROPEAN JOURNAL OF CARDIO-THORACIC SURGERY, SPRINGER VERLAG, BERLIN, DE, Bd. 13, Nr. 2, Februar 1998 (1998-02), Seiten 160-164, XP000993052 ISSN: 1010-7940
- SODIAN R ET AL: "NEW PULSATILE BIORECTOR FOR FABRICATION OF TISSUE-ENGINEERED PATCHES" JOURNAL OF BIOMEDICAL MATERIALS RESEARCH, WILEY, NEW YORK, NY, US, Bd. 58, Nr. 4, 2001, Seiten 401-405, XP001037742 ISSN: 0021-9304

## Beschreibung

Jedes Jahr versterben alleine in den USA ca. 20.000 Patienten an den Folgen einer Herzklappendysfunktion, und mehr als 60.000 Patienten sind wegen einer bereits erkannten Dysfunktion gezwungen, ein oder mehrere Herzklappen operativ ersetzen zu lassen. Als Ersatz für die eigene Herzklappe kommen entweder mechanische oder biologische Klappenprothesen (Xenografts) in Frage, seltener werden kryopreservierte oder glutaraldehydfixierte Homografts verwendet.

Mechanische Klappenprothesen führen jedoch oft zu Fremdkörperreaktionen mit thromboembolischen Komplikationen, die durch die mit der künstlichen Herzklappe veränderten Strömungsverhältnisse im Herzen begünstigt werden. Daher ist eine lebenslange Antikoagulation des betroffenen Patienten erforderlich, die zu einer permanent erhöhten Blutungsgefahr führt. Eine weitere, oft lebensbedrohliche Komplikation bei Patienten mit einer mechanischen Herzklappe sind Infektionen.

Bei den Xenografts handelt es sich meistens um Schweineklappen, die mit Glutaraldehyd behandelt sind. Schweineklappenprothesen können mit guten Ergebnissen bei älteren Patienten eingesetzt werden, neigen aber zur Degeneration nach nur ca. 12 bis 15 Jahren, so dass sie für junge Leute in der Regel nicht in Frage kommen. Weiterhin besteht bei Schweineklappenprothesen im Vergleich zum gesunden Herzen eine erhöhte Infektionsgefahr. Darüber hinaus neigen Schweineklappen zur Kalzifizierung, weshalb sie zum Einsatz bei Kindern und jungen Leuten, die einen erhöhten Calciumstoffwechsel aufweisen, ungeeignet sind. Schließlich stellen sie ebenfalls körperfremdes Gewebe dar, das mit einer gewissen Wahrscheinlichkeit vom körpereigenen Immunsystem als fremd erkannt wird und damit adversive Immunprozesse auslösen kann.

Als dritte Möglichkeit stehen Homografts, d. h. aus humanen Spendern isolierte, fixierte Herzklappen, zur Verfügung. Homografts sind zwar gegen Infektionen relativ resistent, stellen jedoch ebenfalls körperfremdes Gewebe dar, das mit einer gewissen Wahrscheinlichkeit Immunreaktionen hervorruft. Darüber hinaus neigen Homografts ebenso wie Schweineklappenprothesen zur Kalzifizierung und unterliegen daher einer erheblichen Degeneration, die in der Regel eine Reoperation nach 7 bis 12 Jahren erforderlich macht. Homografts stehen darüber hinaus in nur äußerst begrenztem Umfang zur Verfügung.

Neben den bereits beschriebenen Nachteilen der bisher als Klappenersatz verwendeten Klappenprothesen, d. h. der Auslösung von Immunreaktionen, der erhöhten Infektionsgefahr, der Gefahr thromboembolischer Prozesse und der Degenerationsneigung, ist allen bisher bekannten Klappen gemeinsam, dass sie aus anorganischem Material oder fixiertem organischen Material bestehen und ihnen daher wichtige Eigenschaften lebender Matrix, z. B. die Fähigkeit zu Reparationsprozessen, zur Rekonfiguration oder zum Wachstum fehlen. Daraus folgt u. a., dass bei kindlichen Klappenpatienten bisher regelmäßig Reoperationen in Kauf genommen werden mussten. Zusätzlich zu dem jeder Herzoperation inhärenten Risiko steigt jedoch mit jeder Reoperation das Morbiditäts- und Mortalitätsrisiko, da durch die vorangegangenen Operationen erhebliche Verwachsungen im Thorax auftreten.

Es besteht daher ein dringender Bedarf für einen Herzklappenersatz, der die zuvor beschriebenen Nachteile vermeidet. Zu diesem Zweck ist bereits vorgeschlagen worden, künstliche Herzklappen durch "Tissue Engineering" herzustellen. Das "Tissue Engineering" befasst sich mit der Entwicklung "biohybrider" Implantate, die im Körper zu Geweben oder gar zu ganzen Organsystemen heranwachsen. Auch die Herstellung biohybrider Herzklappen in Form von einzelnen Klappensegeln ist bereits beschrieben worden; die durch "Tissue Engineering" hergestellten Herzklappensegel hatten jedoch bisher den Nachteil, dass sie inadäquate, nicht ausreichende bindegewebige Strukturen aufwiesen und daher den im Herzen herrschenden Strömungsverhältnissen nach Auflösung der biodegradablen Trägerstruktur nicht hätten standhalten können.

DE 19919625 beschreibt ein *in vitro*-Verfahren zum Herstellen einer homologen Herzklappe. Die dort beschriebene Herzklappe baut auf einem biologisch abbaubaren Träger auf, der m it homologen Fibroblasten und/oder Myofibroblasten zur Ausbildung einer bindegewebsähnlichen Matrix inkubiert und dann mit Endothelzellen besiedelt wird. Die bindegewebsähnliche Matrix wird dann anschließend zur Gewebereifung in einen Bioreaktor überführt. Diese Herzklappe ist den Stömungsverhältnissen im menschlichen Körper bestens angepasst. Die in DE 19919625 beschriebene Herzklappe besteht zum Zeitpunkt ihrer Implantation fast vollständig aus autologem Zellmaterial, das dann in das Empfängerherz eingenäht wird. Ein Nachteil dieser Herzklappe könnte unter Umständen sein, dass die chirurgische Implantation technisch schwierig durchführbar ist. Außerdem könnte ein Problem entstehen, wenn die Naht durch das einzunähende autologe, tissue-engineerte Gewebe geführt werden muss. Aufgrund der extrem hohen Belastung, der die Herzklappe anschließend im menschlichen Körper ausgesetzt ist, könnte es im Bereich der Naht zu Rissen kommen.

Aufgabe der Erfindung ist es daher, verbesserte homologe Herzklappen sowie ein Verfahren zu ihrer Herstellung zur Verfügung zu stellen.

Erfindungsgemäß wird die Aufgabe durch ein *in vitro*-Verfahren zum Herstellen einer homologen Herzklappe gelöst, das die folgenden Schritte umfasst:
- Bereitstellen eines biologisch abbaubaren Trägers (Scaffold),
- Besiedeln des Trägers mit homologen Fibroblasten und/oder Myofibroblasten zur Ausbildung einer bindegewebigen Matrix,
- ggfls. Besiedeln der bindegewebigen Matrix mit Endothelzellen
- Befestigen der bindegewebigen Matrix auf einer nicht oder schwer abbaubaren Rahmenkonstruktion (Stent),
wobei die ggfls. mit Endothelzellen besiedelte bindegewebige Matrix vor oder nach der Befestigung auf der Rahmenkonstruktion in eine pulsatile Flusskammer, in der sie steigenden Flussraten ausgesetzt werden kann, eingebracht wird, und die Flussrate kontinuierlich oder diskontinuierlich erhöht wird.

In einem alternativen Verfahren wird eine homologe Herzklappe hergestellt durch
- Bereitstellen eines biologisch abbaubaren Trägers (Scaffold), der mit einer nichtabbaubaren Rahmenkonstruktion (Stent) fest verbunden ist,
- Besiedeln des Trägers mit homologen Fibroblasten und/oder Myofibroblasten zur Ausbildung einer bindegewebigen Matrix,
- ggfls. Besiedeln der bindegewebigen Matrix mit Endothelzellen
- Einbringen der Rahmenkonstruktion mit der damit verbundenen bindegewebigen Matrix in eine pulsatile Flusskammer, in der sie steigenden Flussraten ausgesetzt werden kann,
- kontinuierliches oder diskontinuierliches Erhöhen der Flussrate.

Mit den erfindungsgemäßen Verfahren können homologe Herzklappen hergestellt werden, die sämtliche Vorteile der aus der DE19919625 bekannten Herzklappe aufweisen und überdies vermeiden, dass zum Zeitpunkt der Implantation der Klappe eine Naht durch die bindegewebigen Strukturen der Herzklappe geführt werden muss. Sie sind den im Körper herrschenden Stömungsverhältnissen gewachsen und einfach chirurgisch implantierbar.

Die Verfahren zum Herstellen der erfindungsgemäßen Herzklappe sowie die dadurch hergestellte Herzklappe sollen im Folgenden näher erläutert werden.

In der folgenden Beschreibung bedeutet der Terminus "Träger" eine azelluläre Struktur, die, wie unten genauer erläutert wird, entweder aus synthetischen Fasern oder einem azellulären Bindegewebsgerüst gebildet ist. Der Begriff "Matrix" bezeichnet eine bindegewebige Struktur, die neben Fibroblasten und Myofibroblasten typische Bestandteile einer Extrazellulärmatrix, nämlich Kollagen, Elastin und Glycosaminoglycane enthält. Mit Matrix bezeichnete Strukturen enthalten typischerweise im Abbau begriffene Trägerbestandteile oder gar keine Trägerbestandteile mehr.

Zur Durchführung des erfindungsgemäßen Verfahrens wird zunächst ein biologisch abbaubarer Träger bereitgestellt. Das Trägermaterial soll dabei einerseits eine gewisse Zeitlang stabil sein, um eine ausreichende Besiedlung bzw. Durchdringung mit Fibroblasten und/oder Myofibroblasten zu ermöglichen und die Ausbildung einer bindegewebigen Matrix erreichen zu können, andererseits innerhalb einer vertretbaren Zeit, die idealerweise kleiner ist, als die Zeit, die die Bildung der homologen Klappenprothese in Anspruch nimmt, insgesamt aufgelöst werden können. Es ist bevorzugt, dass der Abbau nach ca. 8 Tagen beginnt; er sollte in der Regel in weniger als 3 Monaten, bevorzugt schon nach 4 bis 6 Wochen abgeschlossen sein.

Nach Ausbildung einer soliden bindegewebigen Matrix-Struktur, deren abbaubarer Träger noch nicht aufgelöst zu sein braucht, wird diese ggfls. mit Endothelzellen besiedelt. Nach erfolgter Besiedelung wird die bindegewebige Matrix auf eine nicht oder schwer abbaubare Rahmenkonstruktion aufgebracht. Alternativ kann jedoch ein bereits mit einer Rahmenkonstruktion fest verbundener Träger den Besiedelungsschritten unterworfen werden. Die möglichen alternativen Verfahrensvarianten sollen im Folgenden näher beschrieben werden.

In einer Variante des erfindungsgemäßen Verfahrens wird der biologisch abbaubare Träger (Scaffold) zunächst mit homologen Fibroblasten und/oder Myofibroblasten zur Ausbildung einer bindegewebigen Matrix besiedelt werden. Anschließend wird die Matrix ggfls. mit Endothelzellen besiedelt. Erfindungsgemäß kann die vorgebildete herzklappenanaloge Struktur nun in einem weiteren Verfahrensschritt zur Gewebereifung und Optimierung der hämodynamischen Funktion in eine pulsatile Flusskammer eingebracht werden, in der sie steigenden Flussraten ausgesetzt werden kann. Durch kontinuierliches oder diskontinuierliches Erhöhen der Flussrate wird sie dabei an die Strömungsverhältnisse im menschlichen Körper adaptiert. Zur weiteren Stabilisierung wird die herzklappenanaloge Struktur auf einer biokompatiblen Rahmenkonstruktion aus nicht oder schwer abbaubarem Material befestigt, die ggfls. nochmals in die pulsatile Flusskammer eingesetzt wird. In dem Fall, dass nach Befestigung auf der Rahmenkonstruktion in der Flusskammer an die Strömungsbedingungen im menschlichen Herzen adaptiert wird, kann die erste Inkubation in der pulsatilen Flusskammer ausgelassen werden. Durch diese Verfahren werden vitale Herzklappenprothesen erhalten, die den Strömungsverhältnissen im menschlichen Körper gewachsen sind.

In einer alternativen Variante des erfindungsgemäßen Verfahrens kann der biologisch abbaubare Träger (Scaffold) mit der nicht oder schwer abbaubaren Rahmenkonstruktion (Stent) bereits vor der Besiedelung fest verbunden werden. In einem weiteren Schritt wird dann der mit der Rahmenkonstruktion verbundene Träger zur Ausbildung einer bindegewebigen Matrix mit homologen Fibroblasten und/oder Myofibroblasten und anschließend ggfls. mit Endothelzellen besiedelt. Zur Gewebereifung und Optimierung der hämodynamischen Funktion wird die vorgebildete herzklappenanalogen Struktur anschliessend in eine pulsatile Flusskammer eingebracht, in der sie steigenden Flussraten ausgesetzt werden kann. Durch kontinuierliches oder diskontinuierliches Erhöhen d er F lussrate wird dabei ebenfalls eine vitale Herzklappenprothese erhalten, die den Strömungsverhältnissen im menschlichen Körper gewachsen ist.

Insgesamt ergeben sich also die folgenden Verfahrensvarianten:
Variante 1:
   - Bereitstellen eines Trägers ohne Rahmenkonstruktion
   - Besiedeln
   - Adaptieren in einer pulsatilen Flusskammer
   - Befestigen auf einer nicht oder schwer abbaubaren Rahmenkonstruktion (Stent)
   - ggfls. Nachadaptieren der "gestententen" Herzklappe
Variante 2:
   - Bereitstellen eines Trägers ohne Rahmenkonstruktion
   - Besiedeln
   - Befestigen auf einer nicht oder schwer abbaubaren Rahmenkonstruktion (Stent)
   - Adaptieren der "gestententen" Herzklappe
Variante 3:
   - Bereitstellen eines Trägers auf einer nicht oder schwer abbaubaren Rahmenkonstruktion
   - Besiedeln
   - Adaptieren

Bei dem Trägermaterial handelt es sich bevorzugt um eine aus Polymerfasern aufgebaute Struktur, um eine poröse Polymerstruktur oder ein azelluläres biologisches Gewebe. Geeignete synthetische Polymere für diese Verwendung schließen bioerodierbare Polymere mit ein, wie z. B. Polyglycolsäure (PGA), Polymilchsäure (PLA)' Polyhydroxyalkanoat (PHA) und Poly-4-Hydroxybutyrat (P4HB), Polycaprolactone (PLGA), Polycarbonate, Polyamide, Polyanhydride, Polyaminosäuren, Polyorthoester, Polyacetate, Polycyanoacrylate sowie abbaubare Polyurethane und nicht erodierbare Polymere wie Polyacrylate, Ethylenvinylacetat-Polymere und andere substituierte Zelluloseacetate sowie Derivate davon. Polyester werden hierbei bevorzugt.

Bevorzugte biologisch abbaubare Polymere, die aus umfassen Polymere folgender Gruppe ausgewählt sind: Polyester der Hydroxycarboxysäuren, Polyanhydride der Dicarboxyester, und Copolymere d er H ydroxycarboxysäuren und der Dicarboxyester.

In einer weiteren Ausführungsform besteht das Material aus einem synthetischen Polymer aus mindestens einem der folgenden Monomere: Glykolid, Laktid, p-Dioxanon, Caprolacton, Trimethylencarbonat, Butyrolacton. In besonderen Ausführungsformen wird das Material ausgewählt aus einer Gruppe bestehend aus Polymeren oder Copolymeren von Glycolsäure, Milchsäure und Sebacinsäure. Polyglykolsäurepolymere werden hierbei bevorzugt.

Diese Polymere können sowohl rein als auch in Mischungen aus zwei oder mehreren der genannten Substanzen oder Mischungen dieser Substanzen mit weiteren biologisch abbaubaren Polymeren verwendet werden. In einer bevorzugten Ausführungsform wird ein Mischpolymer aus 85% PGA und 15% PLA verwendet.

In einer weiteren besonderen Ausführungsform wird der Träger wird aus einem Polyhydroxyalkanoat (PHA) hergestellt. PHA kann dabei mit einem weiteren nichtdegradierbaren Polymer beschichtet werden. Ein bevorzugtes Polyhydroxyalkanoat für diese Verwendung degradiert *in vivo* innerhalb von weniger als 9 Monaten, noch bevorzugter in weniger als 6 Monaten und am stärksten bevorzugt in weniger als 3 Monaten. Eine bevorzugte Zusammensetzung der Polyhydroxyalkanoate beinhaltet 2- 3-, 4- oder 5-Hydroxysäuren, z. B. Poly-4-hydroxybutyrate. Weiterhin kann die Zusammensetzung ein Poly-4-hydroxybutyrat-co-3-hydroxybutyrat sowie Kombinationen davon beinhalten. Am stärksten bevorzugt wird dabei Poly-4-hydroxybutyrat.

In einer weiteren besonderen Ausführungsform besteht der Träger aus Homopolymeren und Copolymeren mit einer beliebigen Kombination folgender Monomere: 3-Hydroxybutyrate, 3-Hydroxyvalerat, 3-Hydroxypropionat, 2-Hydroxybutyrat, 4-Hydroxybutyrat, 4-Hydroxyvalerat, 3-Hydroxyhexanoat, 3-Hydroxyheptanoat, 3-Hydroxyoctanoat, 3-Hydroxynonanoat, 3-Hydroxytridecanoat, 3-Hydroxytetradecanoat, 3-Hydroxypentadecanoat, 3-Hydroxyhexadecanoat, 3-Hydroxyheptadecanoat und 3-Hydroxyoctadecanoat.

Es hat sich als sinnvoll erwiesen, biologisch abbaubare Träger mit einer Polymerdichte von ca. 40 bis 120 mg/cm³ zu verwenden. Unterhalb von 40 mg/cm³ ist das Polymergewebe zu labil, oberhalb von 1 20 m g/cm³ ist das Gewebe zu dicht, um das Eindringen von Fibroblasten innerhalb eines vertretbaren Zeitraums zuzulassen. In bevorzugten Ausführungsformen beträgt die Dichte des biologisch abbaubaren Trägers 50 bis 80 mg/cm³, besonders bevorzugt 70 mg/cm³. In der vorliegenden Erfindung wurde mit guten Ergebnissen ein polymerer Träger der Fa. Albany International Research, Mensville, MA, USA, mit einer Dichte von ca. 70 mg/cm³ verwendet, sowie ein polymerer Träger der Fa. TRANSOME INC., Palm Bay, FL. USA.

Die Fasern des Trägers können einen Durchmesser von 6 bis 20 µm haben, bevorzugt 10 bis 18 µm. Es sind jedoch auch Gewebe mit anderen Faserstärken denkbar, die jedoch einerseits dem Träger eine gewisse Stabilität verleihen müssen, andererseits die Besiedelung und Durchdringung des Trägers mit Fibroblasten oder Myofibroblasten zulassen müssen. Für poröse (schwammartige) Polymerformen haben sich Porengrößen von 80 - 240 µm als günstig erwiesen. Die Poren können durch die sogenannte "Salt-Leaching" Technik erzielt werden, die dem Fachmann bekannt ist.

Statt eines synthetischen Trägers, wie zuvor beschrieben, ist die Verwendung eines azellulären Bindegewebsgerüstes denkbar. So könnte beispielsweise eine Schweineklappe in ein immunologisch neutrales Gewebe umgewandelt werden (Bader et al., Eur. J.-Cardiothorac. Surg. 14, 279, 1998), das anschliessend mit homologen Zellen besiedelt werden könnte. Auch humane Herzklappen können nach Neutralisierung wieder besiedelt werden.

Der biologisch abbaubare Träger wird zunächst mit einer Fibroblastenpopulation inkubiert. Bei Verwendung homologer Fibroblasten und/oder Myofibroblasten, d. h. von Fibroblasten und/oder Myofibroblasten aus einem Menschen, aber nicht unbedingt dem Patienten, sollte auf gleiche HLA-Typisierung geachtet werden. Fibroblastenpopulationen können dabei z. B. aus peripheren Blutgefäßen, sowohl Arterien als auch Venen, gewonnen werden. Hierzu bietet sich insbesondere die Arteria radialis des Unterarmes an, die wegen der arteriellen Doppelversorgung des Armes in den meisten Fällen zur schadlosen Explantation zur Verfügung steht. Alternativ können Gefäßzellen aus Blutgefäßen des Beines, z. B. der Vena saphena gewonnen werden. Weiterhin können die Myofibroblasten und Endothelzellen aus Knochenmarks-Vorläuferzellen oder aus pluripotenten Stammzellen oder genetisch manipulierten Zellen gewonnen werden.

Die Zellen können beispielsweise aus Gefäßfragmenten gewonnen werden, in dem die Gewebestückchen zunächst, wie in Zünd et a/. (Eur. J. Cardiothorac Surg. 13,160, 1998) beschrieben, in Gewebebruchstücke zerstückelt und ca. 1 bis 2 Wochen unter normalen Zellkulturbedingungen (37°C, 5 CO₂, 95 % Luftfeuchtigkeit) inkubiert werden, bis die Zellen auf dem Boden der Kulturschale eine konfluente Zellschicht bilden. Anschließend werden sie mehrfachen Passagen unterworfen, um eine von restlichem Gewebematerial freie Zellkultur zu erhalten. Nach zwei bis drei Passagen können die gemischten Zellpopulationen gereinigt werden, in dem sie mit einem für Endothelzellen spezifischen Fluoreszenzmarker (Dil-Ac-LDL, von Medical Technologies Inc., Stoughton, MA) inkubiert werden und mittels Durchflußzytometrie (FACStar Plus, Becton Dickinson) getrennt werden. Fluoreszenzmarkierte Zellen sind Endothelzellen, nicht markierte Zellen sind Fibroblasten und Myofibroblasten. Diese werden weitere zwei bis drei Wochen kultiviert und während dieser Zeit zwei bis vier Passagen unterworfen, um eine ausreichende Anzahl an Zellen für die anschließende Besiedelung des Trägers zu erhalten.

Eine wie beschrieben gereinigte oder jede andere reine Fibroblasten/Myofibroblastenkultur kann nunmehr zur Besiedelung des Polymerträgers eingesetzt werden. Dazu werden pro Quadratzentimeter Oberfläche des Trägers ca. 10⁵ bis 6 x 10⁸ Fibroblasten und/oder Myofibroblasten eingesetzt. Unter "Oberfläche" ist in diesem Fall nicht die tatsächliche Oberfläche des Polymers, sondern die bei Betrachtung des Trägers von oben in einer Ebene erkennbare Fläche gemeint. Üblicherweise wird den Fibroblasten 60 bis 90 min. Zeit gegeben, um sich an den Träger anzuheften. Anschließend kann das überstehende Medium entfernt werden und ein weiteres Mal Fibroblastensuspension zugegeben werden. Idealerweise lässt man jedoch zwischen der ersten und zweiten Zugabe von Fibroblastensuspension 2 bis 36 Stunden, bevorzugt 24 Stunden verstreichen.

In einer bevorzugten Ausführungsform des erfindungsgemäßen Verfahrens werden dem Träger bzw. sich der nach der erstmaligen Fibroblastenzugabe allmählich ausbildenden Matrix weitere 3 bis 14 mal, besonders bevorzugt 5 bis 10 mal Fibroblasten und/oder Myofibroblasten zugefügt.

Unter den üblicherweise für das Zellwachstum von Fibroblasten verwendeten Bedingungen (z. B. 5 % CO₂, Inkubation bei 37°C, steriles Medium) entwickelt sich nach ca. ein bis drei Wochen eine solide bindegewebige Struktur. In einer bevorzugten Ausführungsform wird diese Struktur anschließend mit einer reinen Endothelzeli-Suspension inkubiert. Die Endothelzellen können genauso wie die Fibroblasten durch FACS angereichert und anschließend in mehreren Passagen (bevorzugt 3) expandiert werden. Auch für Endothelzellen ist es bevorzugt, die Besiedelung mit jeweils ca. 10⁵ bis 5x10⁸ Endothelzellen mehrfach zu wiederholen, z. B. 3 bis 14 mal. In bevorzugten Ausführungsformen wird die Besiedelung mit Endothelzellen 5 bis 10 mal wiederholt. Zwischen zwei Besiedelungsschritten sollten mindestens 60 min., bevorzugt jedoch 2 bis 24 Stunden liegen. Der Endothelzellbesiedelungsschritt ist jedoch optional.

Bei den zum Besiedeln des Trägers verwendeten Zellen handelt es sich bevorzugt um humane Zellen. Besonders bevorzugt ist es jedoch, autologe Fibroblasten und/oder Myofibroblasten sowie ggfls. Endothelzellen zu verwenden. Dazu wird dem Patienten, dessen Herzklappe ersetzt werden soll, Gewebe z. B. aus einem seiner Gefäße entnommen. Wie oben bereits erwähnt, bieten sich dazu die Arteria radialis sowie die Vena saphena oder Knochenmark an. Die Verwendung der autologen Zellen zur Konstruktion der Herzklappe hat den wesentlichen Vorteil, dass die Klappe nach Implantation in den Patienten kein körperfremdes Gewebe darstellt und somit Immunreaktionen gegen die künstliche Herzklappe so gut wie ausgeschlossen erscheinen.

Ca. 14 Tage nach der optionalen Zugabe der Endothelzellen lässt sich histologisch und immunhistochemisch ein Gewebe mit einem superfiziellen Einzelzellschicht aus Endothelzellen und einer bindegewebigen Grundstruktur nachweisen.

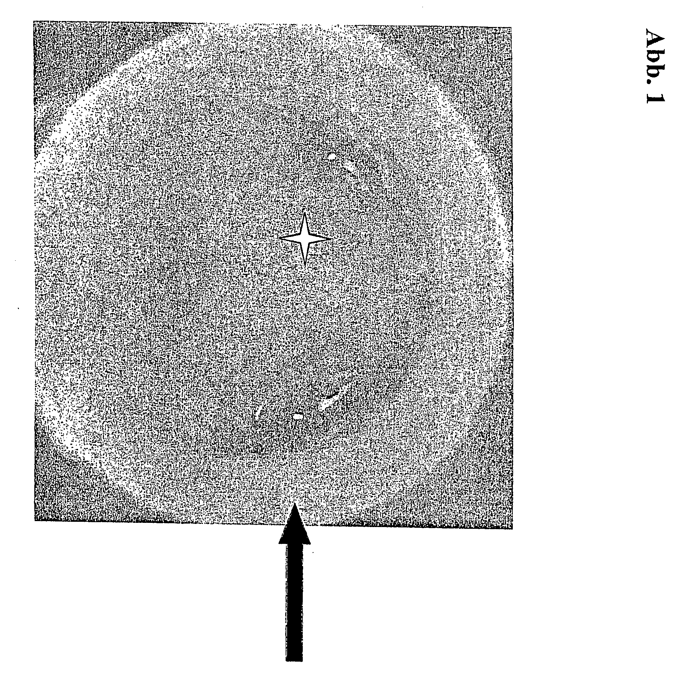

In einer bevorzugten Ausführungsform hat die bindegewebige Matrix die Form einer Herzklappe und ist mit einem breiten bindegewebigen Rand, dem sogenannten Nahtring versehen, der auf einer kreisförmigen Rahmenkonstruktion fixiert wird. Ein Beispiel für eine solche Herzklappe mit Nahtring ist in Abbildung 1 gezeigt.

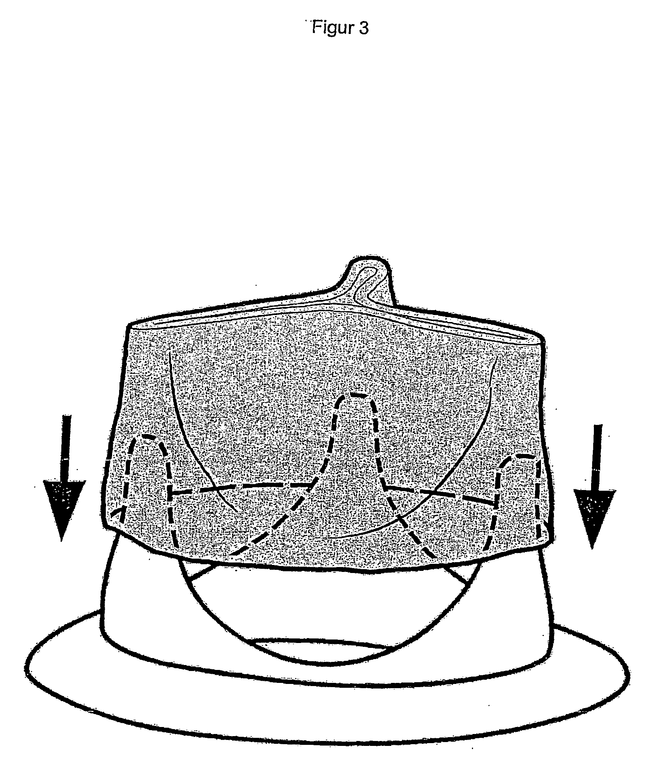

In einer weiteren bevorzugten Ausführungsform hat die bindegewebige Matrix die Form eines Bandes oder eines Ringes. Diese Ausführungsform erfordert einen Nahtring, der mit einer dreizipfeligen Stützstruktur versehen i st, als Rahmenkonstruktion. Das B and bzw. der Ring wird dann um diese dreizipfelige Struktur herumgeführt. Ein Beispiel für diese Ausführungsform ist in Abbildung 3 dargestellt. Der Durchmesser der Rahmenkonstruktion ist dabei individuell wählbar und richtet sich nach den anatomischen Erfordernissen des Patienten.

Die erfindungsgemäße Rahmenkonstruktion (Stent) kann aus verschiedenen Materialien aufgebaut sein. Um dem neu hergestellten Gewebe eine möglichst lange Lebensdauer und Festigkeit zu verleihen, sollte das Material aus nicht abbaubarem biokompatiblem oder alternativ aus schwer abbaubarem biokompatiblem Material aufgebaut sein, z. B. Karbon, PTFE, Dacron, Metall, PHA, bevorzugt Poly-3-Hydroxbutyrat (P3HB). "Schwer abbaubar" bedeutet dabei eine Abbaudauer von mehr als einem Jahr.

Die Befestigung der bindegewebigen Matrix auf der Rahmenkonstruktion kann durch konventionelle Nahttechnik erfolgen. In einer Ausführungsform kann die Matrix mittels Fibrinkleber auf der Rahmenkonstruktion fixiert werden. Besonders bevorzugt erfolgt die Befestigung der bindegewebigen Matrix auf dem Träger durch konventionelle Nahttechnik in Verbindung mit Fibrinkleber. Die Form der einzelnen Herzklappensegel kann ebenso entweder durch eine Naht oder durch Verkleben mit Fibrinkleber stabilisiert werden, in dem die über die Zipfelspitzen in Richtung des Kreismittelpunktes umlaufenden Ränder vernäht bzw. verklebt werden.

Erfindungsgemäß kann nun in einem weiteren Verfahrensschritt die vorgebildete herzklappenanaloge Struktur in eine pulsatile Flusskammer eingebracht werden, in der sie steigenden Flussraten ausgesetzt werden kann. Es wurde festgestellt, dass durch eine langsame Adaptation der Flussraten die Bildung einer strömungsbeständigen bindegewebigen Matrix erreicht werden kann.

Zum Durchführen des erfindungsgemäßen Verfahrens eignet sich z. B. der in der DE19919625 beschriebene Bioreaktor.

In einer Ausführungsform der Erfindung werden Flussraten zwischen 5 ml/min. und 8000 ml/min., bevorzugt zwischen 30 ml/min. und 5 000 ml/min, besonders bevorzugt 50 ml/min. bis 2000 ml/min verwendet. Die Angaben beziehen sich auf den Fluss durch die Klappenprothese. Als anfängliche Flussrate haben sich Flussraten von 50 bis 100 ml/min. als geeignet erwiesen. Diese Flussraten werden z. B. mit einer Pulsfrequenz von 5 bis 10 Pulsen pro Minute durch die Herzklappe geschickt. Die Flussrate wird anschließend kontinuierlich oder diskontinuierlich auf bis zu 5000 ml/min. gesteigert. Gleichzeitig wird die Pulsfrequenz auf bis zu 180 Pulse/min. angehoben. Bei den angegebenen Daten handelt es sich um die Grenzwerte, die normalerweise nicht überschritten werden.

In bevorzugten Ausführungsformen wird die Flussrate bis auf 2 000 ml/min gesteigert, während die Pulsfrequenz auf 70 bis 100, bevorzugt 80 Pulse/min. angehoben wird. Die Belastung der sich stabilisierenden Herzklappe wird damit nahezu physiologischen Verhältnissen angepasst. Es hat sich als günstig, aber nicht notwendig erwiesen, die Flussrate und die Pulsfrequenz jeweils nach ca. 24 bis 48 Stunden zu steigern. So kann beispielsweise, ausgehend von einer Flussrate von 50 bis 100 ml/min und einer Pulsrate von 5 bis 10 Pulsen/min. am Tag 1 des Aufenthaltes in der pulsatilen Flusskammer, am Tag 3 eine Erhöhung auf 300 ml/min bei 20 bis 25 Pulsen/min, am Tag 5 auf 700 ml/min. und 35 bis 45 Pulsen/min, am Tag 7 auf 1000 ml/min und 50 bis 60 Pulsen/min, am Tag 9 auf 1300 ml/min und 70 bis 80 Pulsen/min., am Tag 11 auf 1500 ml/min. und ca. 100 Pulsen/min, am Tag 13 auf 1750 ml/min und ca. 120 Pulsen/min und am Tag 1 5 auf 2 000 ml/min und 140 Pulsen/min vorgesehen werden. Je nach zur Verfügung stehender Zeit, Größe der Klappe, Größe und Alter des Patienten etc., kann jedoch eine sehr viel langsamere Steigerung der Flussraten sowie der Pulsfrequenz oder die Steigerung auf höhere Flussraten und Pulsfrequenzen sinnvoll sein.

In einer Ausführungsform der Erfindung werden die in der pulsatilen Flusskammer herrschenden systemischen Drücke auf 10 bis 2 40 mmHg eingestellt. Bevorzugt sind systemische Drücke von 60 bis 140, besonders bevorzugt sind systemische Drücke von 80 bis 120 mm Hg.

Die mittels des erfindungsgemäßen Verfahrens hergestellte homologe bzw. autologe Herzklappe weist gegenüber den herkömmlichen mechanischen und biologischen Herzklappen wesentliche Vorteile auf. So besteht die erfindungsgemäße Herzklappe in ihrer bevorzugten Ausführungsform aus autologen Gewebe, d. h. aus Gewebe des zur Herzklappenoperation anstehenden Patienten, sowie aus einem weiter stabilisierenden biokompatiblem Material, das als Rahmenkonstruktion verwendet wird. Dadurch wird eine Fremdkörperreaktion des Klappenempfängers auf das Implantat vermieden. Die Infektionsgefahr bei Empfängern einer erfindungsgemäßen Herzklappe ist somit erheblich reduziert. Eine Antikoagulationstherapie ist nicht erforderlich; damit entfällt die Gefahr hemorrhagischer Komplikationen. Der bei weitem überzeugendste Vorteil der erfindungsgemäßen Herzklappe ist jedoch die Tatsache, dass sie lebendes Gewebe darstellt und daher nach Implantation zu permanenter Regeneration und Reparation befähigt ist. Weiterhin vereint die erfindungsgemäße Herzklappe in ihrer bevorzugten Ausführungsform die Vorteile einer vollkommen autologen Herzklappenprothese und die sehr guten hämodynamischen Funktionen synthetischer Herzklappenprothesen. Letztendlich sind bei erfindungsgemäßen Herzklappen durch die Verwendung der biokompatiblen Rahmenkonstruktion auch bei längerer Verwendung deutlich weniger degenerative Veränderungen und/oder Dysfunktionen zu erwarten, was die Lebensdauer der Herzklappe deutlich erhöht und damit das Risiko einer Reoperation deutlich heruntersetzt.

Die erfindungsgemäße Herzklappe enthält eine bindegewebige innere Struktur, die neben Fibroblasten und Myofibroblasten im Wesentlichen Bestandteile einer normalen extrazellulären Matrix, nämlich Kollagen, Elastin und Glycosaminoglykane enthält. Damit weisen die erfindungsgemäßen Klappen einen der nativen Klappe bzw. dem nativen Klappensegel entsprechenden Anteil an Kollagen (26-60 %), Elastin (2-15 %) und Glycosaminoglycanen auf. Diese auf einem biologisch abbaubaren Träger (Scaffold) aufgebaute und mit Endothelzellen besiedelte bindegewebige innere Struktur wird durch eine biokompatible Rahmenkonstruktion weiter stabilisiert. Die Befestigung der bindegewebigen Struktur auf der biokompatiblen Rahmenkonstruktion erfolgt wie oben beschrieben. Eine solche Herzklappenprothese vereint in sich die Vorteile einer autologen Herzklappenprothese und die sehr gute chirurgische Implantierbarkeit und Funktion synthetischer Herzklappenprothesen.

Es konnte gezeigt werden, dass die erfindungsgemäßen Herzklappen Flussraten von mehr als 2000 ml/min., entsprechend den in einem erwachsenen Humanherzen herrschenden Flussverhältnissen, standhalten. Somit kann erfindungsgemäß eine autologe Herzklappe zur Verfügung gestellt werden, die bedingungslos zur Implantation in kindliche wie auch erwachsene Patienten geeignet ist.

Die folgenden Abbildungen und Beispiele erläutern die Erfindung.

Abbildung 1 zeigt einen aus einem Polymer vorgebildeten. Träger (s. Stern) mit Nahtring (s. Pfeil) nach Besiedelung mit Fibroblasten/Myofibroblasten und Endothelzellen.

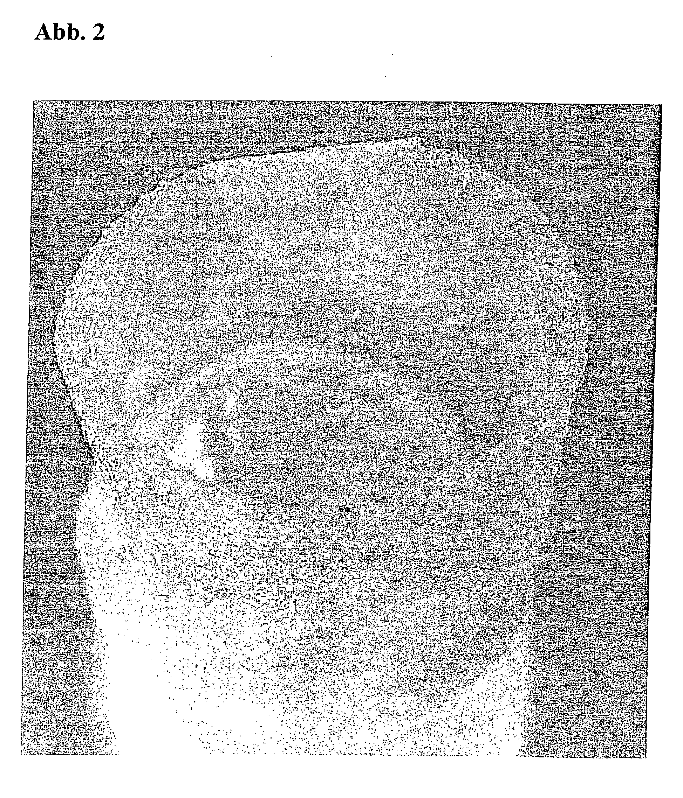

Abbildung 2 zeigt eine röhrenförmige besiedelte Matrix, aus der Ringe von 3,5 cm Breite geschnitten werden können, die um die in Abbildung 3 gezeigte Rahmenkonstruktion gelegt werden.

Abbildung 3 zeigt eine schematische Darstellung der Rahmenkonstruktion aus Abb. 3 mit um die dreizipfelige Stützstruktur geführter besiedelter Matrix.

### Beispiel 1: Herstellung von klappentragenden Conduit-(Röhren) Trägern

Zum Herstellen des klappentragenden Conduit-Trägers wird ein nicht-gewebtes Polyglykolsäurepolymer (Fiberdurchmesser: 12 - 15 p. m, Polymerdichte: 70 mg/ml, Albany International Research, Mansfield MA, USA.) verwendet. Das Polymer wird in der Art zugeschnitten, dass es eine Röhre m it 19 m m Durchmesser bildet. In diesen Conduit werden 3 dreieckige Segel eingefügt. Dieser Träger kann zum Herstellen von 3-segeligen Klappen, d. h. Pulmonal-, Aorten- und Tricuspidalklappen verwendet werden. Für Mitralklappen werden 2 Segel eingefügt.

### Beispiel 2: Herstellung einer dreisegeligen von einer Rahmenkonstruktion stabilisierten Herzklappenprothese

Ein dreisegeliger klappentragender Condiut-Träger wird sterilisiert und in Medium (DM EM, GIBCO BRL-Life Technologies) für 24 Stunden eingelegt, um die Polymeroberfläche einzuweichen. Daraufhin wird der klappenförmige Träger pro Quadratzentimeter Oberfläche mit 4 Mio. Fibroblasten alle 90 Minuten insgesamt 6 mal besiedelt. Weiterhin wird der besiedelte Träger für 2 Wochen inkubiert (5 % CO₂, 37°C, 95 % Luftfeuchtigkeit). Das Medium wird alle 4 Tage unter sterilen Bedingungen gewechselt. Anschließend werden Endothelzellen auf den besiedelten klappenförmigen Träger aufgebracht (3-4 Mio. Endothelzellen pro Quadratzentimeter Oberfläche, 6 Besiedlungen alle 90 Minuten). Nach weiteren 2 Wochen wird das entstandene Gewebe über eine vorbereitete biologisch kompatible Rahmenkonstruktion gestülpt und mit der Rahmenkonstruktion durch eine Naht fest verbunden. Anschließend wird die gesamte Konstruktion in die Flusskammer des Bioreaktors unter sterilen Kautelen eingebracht und hier in Durchflussposition installiert. Der Bioreaktor wird nun mit Medium gefüllt und in den Zellinkubator gestellt. Nachdem über den Druckluftschlauch die Konnektion zur außerhalb des Inkubators stehenden Pumpe hergestellt wurde, wird mit minimalen pulsatilen Flüssen begonnen (50 ml/min). In 2-Tages-Schritten wird die Flussrate und Pulsrate gesteigert auf 100 ml/min (Puls 10), 300 ml (Puls 25), 700 ml (Puls 35), 1000 ml (Puls 60) für insgesamt weiter 4 Tage. Anschliessend (nach 14 Tagen) wird das nun gebildete Gewebe unter sterilen Bedingungen entnommen und zur biochemischen, histologischen und mechanischen Analyse asserviert.

### Beispiel 3: Herstellung von dreisegeligen von einer mehrzipfeligen Rahmenkonstruktion stabilisierten herzklappenförmigen Trägern:

Zur Herstellung des herzklappenförmigen Trägers wird 1-2 mm dickes, nicht gewebtes Ko-Polymer aus Polyglycolsäure (PGA) und Polyhydroxyalkanoat (PHA) (Fiberdurchmesser 12-15 µm, Polymerdichte 70 mg/ml) verwendet und so zugeschnitten, dass ein 3,5 cm breites und 8,0 cm langes Band entsteht. Dieses Band wird an den Endpunkten unter Verwendung von resorbierbaren Nahtmaterial verbunden und zusätzlich an den Überlappungszonen unter Applikation von Hitze (60-70°C) verschweißt. Der nun entstandene Ring wird über eine dreizipfelige Rahmenkonstruktion (Dacron) gestülpt und m it dieser mittels Naht und unter Verwendung von Fibrinkleber verbunden. Nachfolgend wurden die einzelnen Herzklappensegel über das Rahmenkonstrukt wiederum unter Applikation von Hitze in die herzklappentypische, bauchige dreisegelige Form ausgeformt.

### Beispiel 4: Herstellung einer dreisegeligen von einer dreizipfeligen Rahmenkonstruktion stabilisierten Herzklappenprothese

Ein dreisegeliger von einer dreizipfeligen Rahmenkonstruktion stabilisierten Träger wir sterilisiert und in Medium (DM, EM, GIBCO BRL-Life Technologies) für 24 Stunden eingelegt, um die Polymeroberfläche einzuweichen. Darauf hin wird der klappenförmige Träger pro Quadratzentimeter Oberfläche mit 4 Mio. Fibroblasten alle 90 Minuten insgesamt 6 mal besiedelt. Weiterhin wird der besiedelte Träger für 2 Wochen inkubiert (5 % CO₂, 37°C, 95 % Luftfeuchtigkeit). Das Medium wird alle 4 Tage unter sterilen Bedingungen gewechselt. Anschließend werden Endothelzellen auf den besiedelten klappenförmigen Träger aufgebracht (3-4 Mio. Endothelzellen pro Quadratzentimeter Oberfläche, & Besiedelungen alle 90 Minuten). Anschließend wir die gesamte Konstruktion in die Flusskammer des Bioreaktors unter sterilen Kautelen eingebracht und hier in Durchflussposition installiert. Der Bioreaktor wird nun mit Medium gefüllt und in den Zellinkubator gestellt. Nachdem über den Druckluftschlauch die Konnektion zur außerhalb des Inkubators stehenden Pumpe hergestellt wurde, wird mit minimalen pulsatilen Flüssen begonnen (50 ml/min.). In 2-Tages-Schritten wir die Flussrate und Pulsrate gesteigert auf 100 ml/min. (Puls 10), 300 ml (Puls 25, 700 ml (Puls 35), 1000 ml (Puls 60) für insgesamt weitere 4 Tage. Anschliessend (nach 14 Tagen) wird das nun gebildete Gewebe unter sterilen Bedingungen entnommen und zur biochemischen, histologischen und mechanischen Analyse asserviert.

## Patentansprüche

1. *In vitro*-Verfahren zum Herstellen einer homologen Herzklappe, umfassend die folgenden Schritte:
- Bereitstellen eines biologisch abbaubaren Trägers (Scaffold),
- Besiedeln des Trägers mit homologen Fibroblasten und/oder Myofibroblasten zur Ausbildung einer bindegewebigen Matrix,
- ggfls. Besiedeln der bindegewebigen Matrix mit Endothelzellen
- Befestigen der Matrix auf einer nicht oder schwer abbaubaren Rahmenkonstruktion (Stent),
wobei die ggfls. mit Endothelzellen besiedelte bindegewebige Matrix vor und/oder nach Befestigung auf der Rahmenkonstruktion, in eine pulsatile Flusskammer eingebracht wird, in der sie steigenden Flussraten ausgesetzt werden kann, und die Flussrate kontinuierlich oder diskontinuierlich erhöht wird.

2. *In vitro*-Verfahren zum Herstellen einer homologen Herzklappe, umfassend die folgenden Schritte:
- Bereitstellen eines biologisch abbaubaren Trägers (Scaffold), der mit einer nicht oder schwer abbaubaren Rahmenkonstruktion (Stent) fest verbunden ist,
- Besiedeln des Trägers mit homologen Fibroblasten und/oder Myofibroblasten zur Ausbildung einer bindegewebigen Matrix,
- ggfls. Besiedeln der bindegewebigen Matrix mit Endothelzellen
- Einbringen der Rahmenkonstruktion mit der damit verbundenen bindegewebigen Matrix in eine pulsatile Flusskammer, in der sie steigenden Flussraten ausgesetzt werden kann,
- kontinuierliches oder diskontinuierliches Erhöhen der Flussrate.

3. Verfahren nach einem der Ansprüche 1 bis 2, **dadurch gekennzeichnet, dass** der biologisch abbaubare Träger eine biologisch abbaubare Polymermatrix oder eine azelluläre biologische Matrix ist.

4. Verfahren nach einem der Ansprüche 1 b is 3, **dadurch gekennzeichnet, dass** der Träger eine Polyglycolsäure (PGA), Polymilchsäure (PLA), Polyhydroxyalkanoat (PHA), Poly-4-Hydroxybutyrat (P4HB) oder eine Mischung aus zwei oder mehreren dieser Polymere umfasst.

5. Verfahren nach einem der Ansprüche 1 bis 4 , **dadurch gekennzeichnet, dass** der Träger eine Polymerdichte von 40 bis 120 mg/cm³, bevorzugt 50 bis 80 mg/cm³ aufweist.

6. Verfahren nach einem der Ansprüche 1 bis 5, **dadurch gekennzeichnet, dass** der Träger ein poröses Polymer mit einer Porengröße von 80 bis 240 µm ist.

7. Verfahren nach einem d er Ansprüche 1 bis 6, **dadurch gekennzeichnet, dass** die Fasern des Trägers einen Durchmesser von 6 bis 20 µm, bevorzugt 10 bis 18 µm aufweisen.

8. Verfahren nach einem der Ansprüche 1 bis 3 , **dadurch gekennzeichnet, dass** der Träger ein Bindegewebsgerüst einer animalen oder humanen Herzklappe ist.

9. Verfahren nach einem der Ansprüche 1 bis 8, **dadurch gekennzeichnet, dass** der Schritt des Besiedelns mit Fibroblasten und/oder Myofibroblasten 3 bis 14 mal, bevorzugt 5 bis 10 mal wiederholt wird.

10. Verfahren nach einem der Ansprüche 1 bis 9, **dadurch gekennzeichnet, dass** pro Quadratzentimeter Träger/Matrix und Besiedetungsschritt ca. 10⁵ bis 6 x 1₀⁸ Fibroblasten und/oder Myofibroblasten eingesetzt werden.

11. Verfahren nach einem der Ansprüche 1 bis 10, **dadurch gekennzeichnet, dass** der Schritt des Besiedelns mit Endothelzellen 3 bis 14 mal, bevorzugt 5 bis 10 mal wiederholt wird.

12. Verfahren nach einem der Ansprüche 1 bis 11, **dadurch gekennzeichnet, dass** pro Quadratzentimeter Träger/Matrix und Besiedelungsschritt ca. 10⁵ bis 5 x 10⁸ Endothelzellen eingesetzt werden.

13. Verfahren nach einem der Ansprüche 1 bis 12, **dadurch gekennzeichnet, dass** die Fibroblasten und/oder Myofibroblasten und/oder Endothelzellen humane Zellen sind.

14. Verfahren nach einem der Ansprüche 1 bis 13, **dadurch gekennzeichnet, dass** die Fibroblasten und/oder Myofibroblasten und/oder Endothelzellen autologe Zellen sind.

15. Verfahren nach einem der Ansprüche 1 bis 14, **dadurch gekennzeichnet, dass** die Rahmenkonstruktion aus einem biokompatiblem nicht abbaubaren Material besteht.

16. Verfahren nach einem der Ansprüche 1 bis 15, **dadurch gekennzeichnet, dass** die Rahmenkonstruktion aus einem biokompatiblem schwer abbaubaren Material besteht.

17. Verfahren nach einem der Ansprüche 1 bis 16 **dadurch gekennzeichnet, dass** die Befestigung des Trägers auf der Rahmenkonstruktion mittels konventioneller Nahttechnik und/oder Fibrinkleber erfolgt.

18. Verfahren nach einem der Ansprüche 1 bis 17, **dadurch gekennzeichnet, dass** der pulsatilen Flusskammer Flussraten von 5 ml/min bis 8000 ml/min, bevorzugt 50 bis 2000 ml/min eingestellt werden.

19. Verfahren nach einem der Ansprüche 1 bis 18, **dadurch gekennzeichnet, dass** die Flussrate über einen Zeitraum von 1 Woche bis 12 Wochen gesteigert wird.

20. Verfahren nach einem der Ansprüche 1 bis 19, **dadurch gekennzeichnet, dass** die anfängliche Flussrate 50 bis 100 ml/min beträgt.

21. Verfahren nach einem der Ansprüche 1 bis 20, **dadurch gekennzeichnet, dass** die anfängliche Pulsfrequenz 5 bis 10 Pulse/min beträgt.

22. Verfahren nach einem der Ansprüche 1 bis 21, **dadurch gekennzeichnet, dass** die Flussrate bis auf 5000 ml/min gesteigert wird.

23. Verfahren nach einem der Ansprüche 1 bis 22, **dadurch gekennzeichnet, dass** die Pulsfrequenz bis auf 180 Pulse/min gesteigert wird.

24. Verfahren nach einem der Ansprüche 1 bis 23, **dadurch gekennzeichnet, dass** in der pulsatilen Flusskammer systemische Drücke von 10 bis 240 mmHg eingestellt werden.

25. Autologe Herzklappe, **dadurch gekennzeichnet, dass** sie nach einem Verfahren nach einem der Ansprüche 1 bis 24 hergestellt wurde.

## Claims

1. An *in vitro* process for the preparation of a homologous heart valve, comprising the following steps:
• providing a biologically degradable carrier (scaffold),
• populating the carrier with homologous fibroblasts and/or myofibroblasts to form a connective tissue matrix,
• optionally populating the connective tissue matrix with endothelial cells
• attaching the matrix to a non-degradable or poorly degradable frame construction (Stent),
wherein before and/or after attachment to the frame construction, the connective tissue matrix optionally populated with endothelial cells is placed in a pulsatile flow chamber in which it can be subjected to increasing flow rates, and the flow rate is continually or discontinually increased.

2. An *in vitro* process for the manufacture of a homologous heart valve, comprising the following steps:
• providing a biologically degradable carrier (scaffold) which is fixedly connected to a non-degradable or poorly degradable frame construction (Stent),
• populating the carrier with homologous fibroblasts and/or myofibroblasts to form a connective tissue matrix,
• optionally populating the connective tissue matrix with endothelial cells
• placing the frame construction with the connective tissue matrix connected thereto in a pulsatile flow chamber in which it can be subjected to increasing flow rates,
• continually or discontinually increasing the flow rate.

3. The process according to one of claims 1 to 2, **characterized in that** the biologically degradable carrier is a biologically degradable polymer matrix or an acellular biological matrix.

4. The process according to one of claims 1 to 3, **characterized in that** the carrier comprises a polyglycolic acid (PGA), polylactic acid (PLA), polyhydroxyalkanoate (PHA), poly-4-hydroxybutyrate (P4HB) or a mixture of two or more of these polymers.

5. The process according to one of claims 1 to 4, **characterized in that** the carrier has a polymer density of 40 to 120 mg/cm³, preferably 50 to 80 mg/cm³.

6. The process according to one of claims 1 to 5, **characterized in that** the carrier is a porous polymer with a pore size of 80 to 240 µm.

7. The process according to one of claims 1 to 6, **characterized in that** the fibers of the carrier have a diameter of 6 to 20 µm, preferably 10 to 18 µm.

8. The process according to one of claims 1 to 3, **characterized in that** the carrier is a connective tissue scaffold of an animal or human heart valve.

9. The process according to one of claims 1 to 8, **characterized in that** the step of populating with fibroblasts and/or myofibroblasts is repeated 3 to 14 times, preferably 5 to 10 times.

10. The process according to one of claims 1 to 9, **characterized in that** approximately 10⁵ to 6 x 10⁸ fibroblasts and/or myofibroblasts are used per square centimeter carrier/matrix and population step.

11. The process according to one of claims 1 to 10, **characterized in that** the step of populating with endothelial cells is repeated 3 to 14 times, preferably 5 to 10 times.

12. The process according to one of claims 1 to 11, **characterized in that** approximately 10⁵ to 5 x 10⁸ endothelial cells are used per square centimeter carrier/matrix and population step.

13. The process according to one of claims 1 to 12, **characterized in that** the fibroblasts and/or myofibroblasts and/or endothelial cells are human cells.

14. The process according to one of claims 1 to 13, **characterized in that** the fibroblasts and/or myofibroblasts and/or endothelial cells are autologous cells.

15. The process according to one of claims 1 to 14, **characterized in that** the frame construction is made of a biocompatible non-degradable material.

16. The process according to one of claims 1 to 15, **characterized in that** the frame construction is made of a biocompatible, poorly degradable material.

17. The process according to one of claims 1 to 16, **characterized in that** the attachment of the carrier to the frame construction is accomplished by means of conventional suture techniques and/or fibrin glue.

18. The process according to one of claims 1 to 17, **characterized in that** the pulsatile flow chamber is set to flow rates of 5 ml/min to 8000 ml/min, preferably 50 to 2000 ml/min.

19. The process according to one of claims 1 to 18, **characterized in that** the flow rate is increased over a time period of 1 week to 12 weeks.

20. The process according to one of claims 1 to 19, **characterized in that** the initial flow rate is 50 to 100 ml/min.

21. The process according to one of claims 1 to 20, **characterized in that** the initial pulse frequency is 5 to 10 pulse/min.

22. The process according to one of claims 1 to 21, **characterized in that** the flow rate is increased up to 5000 ml/min.

23. The process according to one of claims 1 to 22, **characterized in that** the pulse frequency is increased up to 180 pulse/min.

24. The process according to one of claims 1 to 23, **characterized in that** systemic pressures of 10 to 240 mmHg are set in the pulsatile flow chamber.

25. An autologous heart valve, **characterized in that** it was prepared according to a process according to one of claims 1 to 24.

## Revendications

1. Procédé *in vitro* pour la fabrication d'une valvule cardiaque homologue, comprenant les étapes suivantes :
- mise à disposition d'un support biodégradable (scaffold),
- colonisation du support par des fibroblastes et/ou myofibroblastes homologues pour constituer une matrice de tissu conjonctif,
- éventuellement, colonisation de la matrice de tissu conjonctif par des cellules endothéliales
- fixation de la matrice sur une structure de cadre (stent) non ou difficilement dégradable,
dans lequel la matrice de tissu conjonctif éventuellement colonisée par des cellules endothéliales avant et/ou après fixation sur la structure de cadre, est insérée dans une chambre d'écoulement pulsatile dans laquelle elle peut être soumise à des débits croissants et le débit est augmenté de façon continue ou discontinue.

2. Procédé *in vitro* pour la fabrication d'une valvule cardiaque homologue, comprenant les étapes suivantes :
- mise à disposition d'un support biodégradable (scaffold) qui est fixé à une structure de cadre non ou difficilement dégradable (stent),
- colonisation du support par des fibroblastes et/ou myofibroblastes homologues pour constituer une matrice de tissu conjonctif,
- éventuellement, colonisation de la matrice de tissu conjonctif par des cellules endothéliales
- insertion de la structure de cadre avec la matrice de tissu conjonctif liée à celle-ci dans une chambre d'écoulement pulsatile dans laquelle elle peut être soumise à des débits croissants,
- augmentation continue ou discontinue du débit.

3. Procédé selon l'une quelconque des revendications 1 à 2, **caractérisé en ce que** le support biodégradable comprend une matrice polymère biodégradable ou une matrice biologique acellulaire.

4. Procédé selon l'une quelconque des revendications 1 à 3, **caractérisé en ce que** le support est un acide polyglycolique (PGA), un acide polylactique (PLA), un polyhydroxy-alcanoate (PHA), un poly-4-hydroxybutyrate (P4HB) ou un mélange de deux ou de plusieurs de ces polymères.

5. Procédé selon l'une quelconque des revendications 1 à 4, **caractérisé en ce que** le support présente une densité de polymère de 40 à 120 mg/cm³, de préférence de 50 à 80 mg/cm³_{.}

6. Procédé selon l'une quelconque des revendications 1 à 5, **caractérisé en ce que** le support est un polymère poreux présentant une taille de pores de 80 à 240 µm.

7. Procédé selon l'une quelconque des revendications 1 à 6, **caractérisé en ce que** les fibres du support présentent un diamètre de 6 à 20 µm, de préférence de 10 à 18 µm.

8. Procédé selon l'une quelconque des revendications 1 à 3, **caractérisé en ce que** le support est une structure de tissu conjonctif d'une valvule cardiaque animale ou humaine.

9. Procédé selon l'une quelconque des revendications 1 à 8, **caractérisé en ce que** l'étape de colonisation par des fibroblastes et/ou des myofibroblastes est répétée 3 à 14 fois, de préférence 5 à 10 fois.

10. Procédé selon l'une quelconque des revendications 1 à 9, **caractérisé en ce que** l'on introduit par centimètre carré de support/matrice et par étape de colonisation environ 10⁵ à 5 x 10⁸ fibroblastes et/ou de myofibroblastes.

11. Procédé selon l'une quelconque des revendications 1 à 10, **caractérisé en ce que** l'étape de colonisation par des cellules endothéliales est répétée 3 à 14 fois, de préférence 5 à 10 fois.

12. Procédé selon l'une quelconque des revendications 1 à 11, **caractérisé en ce que** l'on introduit par centimètre carré de support/matrice et par étape de colonisation environ 10⁵ à 5 x 10⁸ cellules endothéliales.

13. Procédé selon l'une quelconque des revendications 1 à 12, **caractérisé en ce que** les fibroblastes et/ou les myofibroblastes et/ou les cellules endothéliales sont des cellules humaines.

14. Procédé selon l'une quelconque des revendications 1 à 13, **caractérisé en ce que** les fibroblastes et/ou les myofibroblastes et/ou les cellules endothéliales sont des cellules autologues.

15. Procédé selon l'une quelconque des revendications 1 à 14, **caractérisé en ce que** la structure de cadre est constituée d'un matériau biocompatible non dégradable.

16. Procédé selon l'une quelconque des revendications 1 à 15, **caractérisé en ce que** la structure de cadre est constituée d'un matériau biocompatible difficilement dégradable.

17. Procédé selon l'une quelconque des revendications 1 à 16, **caractérisé en ce que** la fixation du support sur la structure de cadre s'effectue au moyen d'une technique de suture classique et/ou d'adhésif de fibrine.

18. Procédé selon l'une quelconque des revendications 1 à 17, **caractérisé en ce que** le débit de la chambre d'écoulement pulsatile est ajusté de 5 ml/min à 8000 ml/min, de préférence de 50 à 2000 ml/min.

19. Procédé selon l'une quelconque des revendications 1 à 18, **caractérisé en ce que** le débit est augmenté sur une durée de 1 semaine à 12 semaines.

20. Procédé selon l'une quelconque des revendications 1 à 19, **caractérisé en ce que** le débit initial est de 50 à 100 ml/min.

21. Procédé selon l'une quelconque des revendications 1 à 20, **caractérisé en ce que** la fréquence de pulsation initiale est de 5 à 10 pulsations/min.

22. Procédé selon l'une quelconque des revendications 1 à 21, **caractérisé en ce que** le débit est augmenté jusqu'à 5000 ml/min.

23. Procédé selon l'une quelconque des revendications 1 à 22, **caractérisé en ce que** la fréquence de pulsation est augmentée jusqu'à 180 pulsations/min.

24. Procédé selon l'une quelconque des revendications 1 à 23, **caractérisé en ce que**, dans la chambre d'écoulement pulsatile, la pression systémique est ajustée de 10 à 240 mmHg.

25. Valvule cardiaque autologue, **caractérisée en ce qu'**elle a été fabriquée selon un procédé selon l'une quelconque des revendications 1 à 24.
